# EUROPEAN PATENT APPLICATION

(11) **EP 4 205 731 A1**
(43) Date of publication of application: **05.07.2023**
(21) Application number: 21859483.6
(22) Date of filing: 27.08.2021
(51) Int. Cl.: A61K 9/51, A61K 47/02, A61K 47/42, B01J 13/02, B82Y 5/00, B82Y 40/00

(54) **METHOD FOR RAPIDLY OBTAINING ALBUMIN NANOPARTICLES LOADED WITH MAGNETIC NANOPARTICLES**

(30) Priority: 26.08.2020 CL 20202205
(71) Applicant: Universidad Técnica Federico Santa María, Valparaiso (CL); Universidad De Santiago De Chile, Estacion Central Santiago, 7560873 (CL)
(72) Inventor: VARGAS CANTÍN, Patricio Fernando, Viña del Mar (CL); DÍAZ SALDÍVAR, Patricia Susana, Santiago (CL)
(74) Representative: Ungria López, Javier
(86) International application number: PCT/CL2021/050079
(87) International publication number: WO 2022/040822

(57) **Abstract**

The present invention relates to a method for rapidly obtaining albumin nanoparticles loaded with magnetic nanoparticles, which comprises steps with ultra-high speed agitation. This method allows obtaining said nanoparticles in less than 45 minutes with encapsulation efficiency higher than 90%.

## Description

### Technical field

The present invention relates to the technical field of nanotechnology, particularly to a new method for obtaining nanoparticles. Specifically, the present invention relates to a method for rapidly obtaining albumin nanoparticles loaded with magnetic nanoparticles, which are especially useful in the pharmaceutical industry.

### Background of the Invention

Albumin-based nanoparticles are of special interest in the pharmaceutical industry, since they are biodegradable and biocompatible, and allow transporting different compounds in the organism even in a tissue-specific manner. For this reason, albumin nanoparticles loaded with radiopharmaceuticals or chemotherapeutic agents have been successfully used for the diagnosis and treatment of cancer.

One of the strategies that has been used to transport drugs or chemotherapeutic agents to specific tissue or tumor cells are magnetic albumin-coated nanoparticles. When these magnetic drug-loaded nanoparticles are administered, they accumulate at a certain site with the help of a magnetic field, wherein they release the drug they contain. These nanoparticles are especially useful in the treatment of cancer by magnetic hyperthermia. For example, Kalidasan et al. (2016) report the use of superparamagnetic iron-oxide nanoparticles (SPIONs) and ferromagnetic iron-oxide nanoparticles (FIONs) coated with albumin to improve biocompatibility and specific adsorption rate. This is due to the fact that albumin coating improves colloidal stability, which has an impact on increased heating of the nanoparticles, favoring their use in hyperthermia treatments (Kalidasan V. et al. Bovine Serum Albumin-Conjugated Ferrimagnetic Iron-oxide nanoparticles to Enhance the Biocompatibility and Magnetic Hyperthermia Performance. Nano- Micro Lett. 2016. 8(1):80-93).

In order to obtain this type of nanoparticles, one of the most commonly used methods is desolvation and coacervation. The desolvation method consists of the slow addition of a desolvation agent - such as salts or alcohol, to an aqueous solution of proteins under constant agitation. When the solution reaches a critical concentration of the desolvation agent, proteins associate to form aggregates, which shall be stabilized with a cross-linking agent to form nanoparticles. The solution of aggregates with the cross-linking agent shall be carried out under constant agitation overnight (6-8 hours). The coacervation method is similar to the desolvation method but varies in the number of parameters that affects the process to obtain the desired nanoparticles. For example, some of these parameters include the initial protein concentration, temperature, pH, the concentration and addition rate of the desolvation agent into the protein solution, the concentration of the cross-linking agent, and the rate at which constant stirring is performed (Sundar S. et al. Biopolymeric nanoparticles. Sci. Technol. Adv. Mater. 2010. 11(1):014104; Langer K. et al. Optimization of the preparation process for human serum albumin (HSA) nanoparticles. Int. J. Pharm. 2003. 257:169-180). The variation of any of these parameters directly affects the size of the nanoparticles, the dispersion of the obtained sizes and the encapsulation efficiency of the compounds of interest.

The standard desolvation method is a slow procedure that requires more than 8 hours to obtain stable albumin nanoparticles. For this reason, methods that attempt to accelerate this process are disclosed in the prior art.

An article published by Jahanban-Esfahlan et al. (2016) describes a desolvation method for the preparation of albumin nanoparticles, in which the preparation time is decreased from overnight to three hours. This method consists of adding ethanol as a desolvation agent to an aqueous solution of albumin at a rate of 2 ml/min under constant stirring (1250 rpm) using a 1-cm magnetic stirrer. Then, EDC (N-(3-Dimethylaminopropyl)-N'-ethylcarbodiimide) is used to stabilize the nanoparticles, and kept under constant stirring for three hours under the same conditions previously described (Jahanban-Esfahlan A. et al. A simple improved desolvation method for the rapid preparation of albumin nanoparticles. Int. J. Biol. macromol. 2016. 91 :703-709). Although this process is faster than the standard desolvation method, the use of magnetic stirrers makes this process unsuitable for the preparation of albumin nanoparticles loaded with magnetic nanoparticles, as these nanoparticles are attracted to this stirrer, decreasing their encapsulation efficiency with albumin.

The article published by Wacker et al. (2014) describes the obtaining of iron oxide-loaded albumin nanoparticles by the desolvation method, in which ethanol is used and added to an aqueous solution of albumin and iron-oxide nanoparticles at a rate of 1.5 ml/min under constant agitation (550 rpm). Then, to stabilize the obtained nanoparticles, glutaraldehyde is added to the solution, and they are kept under constant agitation for at least three hours to complete the process (Wacker M. et al. Nanoencapsulation of ultrasmall superparamagnetic particles of iron oxide into human serum albumin nanoparticles. Beilstein J. Nanotechnol. 2014. 5:2259-2266). However, this procedure is still slow and requires at least three hours to obtain stable iron oxide-loaded albumin nanoparticles.

Patent document US 7,875,295 describes a process that decreases the time of the desolvation step to obtain nano or microcapsules of active compounds coated with different polymers. This process uses a high shear mixer to mix the desolvation agent with the aqueous solution of the polymer. In particular, it describes the preparation of nanoparticles using ethanol and an albumin solution, which are mixed at a speed of 5000 rpm. However, this process does not consider the most prolonged step of the method, which corresponds to the stabilization of the albumin nanoparticles with a cross-linking agent; therefore, the duration of this process to obtain such nanoparticles is still extensive.

Consequently, new methodologies are required to quickly obtain albumin nanoparticles loaded with magnetic nanoparticles and with high encapsulation efficiency.

### Summary of the invention

The present invention relates to a method for obtaining albumin nanoparticles loaded with magnetic nanoparticles comprising the steps of:
a) mixing an albumin solution with magnetic nanoparticles and stirring the mixture at 8,000 to 20,000 rpm for 3 to 7 minutes;
b) dropwise adding a desolvation agent to the mixture of step (a) while maintaining a constant agitation at a speed between 8,000 and 20,000 rpm for 1 to 15 minutes;
c) adding a cross-linking agent to the mixture of step (b) while maintaining a constant agitation at a speed between 8,000 and 20,000 rpm for 1 to 15 minutes, and
d) obtaining albumin nanoparticles loaded with magnetic nanoparticles from the mixture of step (c).

In a preferred embodiment of the invention, the magnetic nanoparticles are iron-oxide nanoparticles, preferably superparamagnetic iron-oxide nanoparticles (SPIONs).

In another preferred embodiment of the method of the present invention, the stirring of step (a) is performed at a speed of 15,000 rpm for 5 minutes; the stirring of step (b) is performed at a speed of 15,000 rpm for 5 minutes; and the stirring of step (c) is performed at a speed of 15,000 rpm for 5 minutes.

In another preferred embodiment, the stirring of step (a) is performed with a rotor/stator-type homogenizer; the stirring of step (b) is performed with a rotor/stator-type homogenizer; and the stirring of step (c) is performed with a rotor/stator-type homogenizer.

In another embodiment of the present invention, the albumin solution and the magnetic nanoparticles are mixed at a ratio between 10:1 to 10:5 w/w.

Another embodiment of the method of the present invention additionally comprises a step for adjusting the pH of the step mixture to between 9 and 10.

In a preferred embodiment of the present invention, the desolvation agent is added to the mixture of albumin solution and magnetic nanoparticles at a rate of from 0.1 to 2 ml/min; preferably at a ratio of 0.3 to 0.7 ml per mg of albumin. Preferably, the desolvation agent is selected from the group consisting of ethanol, methanol, isopropanol, and acetone, as well as a combination thereof.

In another preferred embodiment of the present invention, the cross-linking agent is a homobifunctional agent. Preferably, said homobifunctional agent is glutaraldehyde. Preferably, the glutaraldehyde has a concentration at 6.25% and is added at a ratio of 10 µl per mg albumin.

In a preferred embodiment, the albumin nanoparticles loaded with magnetic nanoparticles obtained through this method have a diameter of from 30 to 300 nm.

In one embodiment of the present invention, step (d) additionally comprises a washing step, which preferably is performed with a solution that is selected from distilled water and phosphate buffer saline (PBS).

In another embodiment of the present invention, step (d) additionally comprises a centrifugation step, which preferably is performed at a speed between 8,000 to 20,000 g.

### Brief description of the Figures

FIG. 1 shows the results of the analysis of superparamagnetic iron-oxide nanoparticles (SPIONs). FIG. 1A shows an electron microscopy image of the SPIONs. FIG. 1B shows a graph showing the size (diameter) distribution of such nanoparticles.
FIG. 2 shows the results of the analysis of albumin nanoparticles that were obtained by the conventional desolvation method. FIG. 2A shows an electron microscopy image of said nanoparticles. FIG. 2B shows a graph showing the size (diameter) distribution of said nanoparticles. FIG. 2C shows a graph showing the hydrodynamic size distribution of said nanoparticles.
FIG. 3 shows the results of the analysis of albumin nanoparticles that were obtained by the rapid desolvation method of the present invention. FIG. 3A shows an electron microscopy image of said nanoparticles. FIG. 3B shows a graph showing the size (diameter) distribution of said nanoparticles. FIG. 3C shows a graph showing the hydrodynamic size distribution of said nanoparticles.
FIG. 4 shows the results of the analysis of albumin nanoparticles with 1 mg of SPIONs. FIG. 4A shows an electron microscopy image of said nanoparticles. FIG.4B shows a graph showing the size (diameter) distribution of said nanoparticles. FIG. 4C shows a graph showing the hydrodynamic size distribution of said nanoparticles.
FIG. 5 shows the results of the analysis of albumin nanoparticles with 2 mg of SPIONs. FIG. 5A shows an electron microscopy image of said nanoparticles. FIG. 5B shows a graph showing the size (diameter) distribution of said nanoparticles. FIG. 5C shows a graph showing the hydrodynamic size distribution of said nanoparticles.
FIG. 6 shows the results of the analysis of albumin nanoparticles with 3 mg of SPIONs. FIG. 6A shows an electron microscopy image of said nanoparticles. FIG. 6B shows a graph showing the size (diameter) distribution of said nanoparticles. FIG. 6C shows a graph showing the hydrodynamic size distribution of said nanoparticles.
FIG. 7 shows a graph with the percentage of albumin nanoparticles loaded with SPIONs by virtue of the stirring time of the mixture of the albumin solution and SPIONs with the desolvation agent.
FIG. 8 shows the results of the analysis of the albumin nanoparticles without SPIONs at pH 9 and stirring rate at 15,000 rpm. FIG. 8A shows an electron microscopy image of said nanoparticles. FIG. 8B shows a graph showing the size (diameter) distribution of said nanoparticles. FIG. 8C shows a graph showing the hydrodynamic size distribution of said nanoparticles.
FIG. 9 shows the results of the analysis of the albumin nanoparticles without SPIONs at pH 9 and stirring rate at 20,000 rpm. FIG. 9A shows an electron microscopy image of said nanoparticles. FIG. 9B shows a graph showing the size (diameter) distribution of said nanoparticles. FIG. 9C shows a graph showing the hydrodynamic size distribution of said nanoparticles.
FIG. 10 shows the results of the analysis of albumin nanoparticles loaded with SPIONs at pH 9 and stirring rate at 15,000 rpm. FIG. 10A shows an electron microscopy image of said nanoparticles. FIG. 10B shows a graph showing the size (diameter) distribution of said nanoparticles. FIG. 10C shows a graph showing the hydrodynamic size distribution of said nanoparticles.
FIG. 11 shows the results of the analysis of albumin nanoparticles loaded with SPIONs at pH 9 and stirring rate at 20,000 rpm. FIG. 11A shows an electron microscopy image of said nanoparticles. FIG. 11B shows a graph showing the size (diameter) distribution of said nanoparticles. FIG. 11C shows a graph showing the hydrodynamic size distribution of said nanoparticles.
FIG. 12 shows the results of the analysis of the albumin nanoparticles without SPIONs at pH 10 and stirring rate at 15 000 rpm. FIG. 12A shows an electron microscopy image of said nanoparticles. FIG. 12B shows a graph showing the size (diameter) distribution of said nanoparticles. FIG. 12C shows a graph showing the hydrodynamic size distribution of said nanoparticles.
FIG. 13 shows an electron microscopy image of the result of the synthesis of albumin nanoparticles loaded with SPIONs at pH 10 and stirring rate at 20 000 rpm.
FIG. 14 shows electron microscopy images of the albumin nanoparticles loaded with SPIONs without glutaraldehyde, which were incubated at different pHs for 24 hours. FIG. 14A, 14B, 14C, 14D, and 14E show the nanoparticles at pH 2, 3, 4, 5, and 6, respectively.
FIG. 15 shows size (diameter) scatter plots of albumin nanoparticles loaded with glutaraldehyde-free SPIONs (Na-SPIONs) after 24 h of incubation at different pHs. FIG. 15A, 15B, 15C, 15D, and 15E show the results obtained at pH 2, 3, 4, 5, and 6, respectively.
FIG. 16 shows electron microscopy images of the albumin nanoparticles loaded with SPIONs without glutaraldehyde, which were incubated at different pHs for 48 hours. FIG. 16A, 16B, 16C, 16D, and 16E show the nanoparticles at pH 2, 3, 4, 4, 5, and 6, respectively.
FIG. 17 shows size (diameter) scatter plots of albumin nanoparticles loaded with glutaraldehyde-free SPIONs (Na-SPIONs) after 48 h of incubation at different pHs. FIG. 17A, 17B, 17C, 17D, and 17E show the results obtained at pH 2, 3, 4, 5, and 6, respectively.
FIG. 18 shows electron microscopy images of the albumin nanoparticles loaded with SPIONs without glutaraldehyde, which were incubated at different pHs for 72 hours. FIG. 18A, 18B, 18C, 18D, and 18E show the nanoparticles at pH 2, 3, 4, 5, and 6, respectively.
FIG. 19 shows size (diameter) scatter plots of albumin nanoparticles loaded with glutaraldehyde-free SPIONs (Na-SPIONs) after 72 h of incubation at different pHs. FIG. 19A, 19B, 19C, 19D, and 19E show the results obtained at pH 2, 3, 4, 5, and 6, respectively.
FIG. 20 shows electron microscopy images of the albumin nanoparticles loaded with SPIONs with glutaraldehyde, which were incubated at different pHs for 24 hours. FIG. 20A, 20B, 20C, 20D, and 20E show the nanoparticles at pH 2, 3, 4, 4, 5, and 6, respectively.
FIG. 21 shows size (diameter) scatter plots of albumin nanoparticles loaded with glutaraldehyde-loaded SPIONs (Na-SPIONs + GA) after 24 h of incubation at different pHs. FIG. 21A, 21B, 21C, 21D, and 21E show the results obtained at pH 2, 3, 4, 5, and 6, respectively.
FIG. 22 shows electron microscopy images of the albumin nanoparticles loaded with SPIONs with glutaraldehyde, which were incubated at different pHs for 48 hours. FIG. 22A, 22B, 22C, 22D, and 22E show the nanoparticles at pH 2, 3, 4, 5, and 6, respectively.
FIG. 23 shows size (diameter) scatter plots of albumin nanoparticles loaded with glutaraldehyde-loaded SPIONs (Na-SPIONs + GA) after 48 h of incubation at different pHs. FIG. 23A, 23B, 23C, 23D, and 23E show the results obtained at pH 2, 3, 4, 5, and 6, respectively.
FIG. 24 shows electron microscopy images of the albumin nanoparticles loaded with SPIONs with glutaraldehyde, which were incubated at different pHs for 72 hours. FIG. 24A, 24B, 24C, 24D, and 24E show the nanoparticles at pH 2, 3, 4, 5, and 6, respectively.
FIG. 25 shows size (diameter) scatter plots of albumin nanoparticles loaded with glutaraldehyde-loaded SPIONs (Na-SPIONs + GA) after 72 h of incubation at different pHs. FIG. 25A, 25B, 25C, 25D, and 25E show the results obtained at pH 2, 3, 4, 5, and 6, respectively.

### Detailed description of the Invention

The present invention refers to a new fast, simple, and efficient method for obtaining albumin nanoparticles loaded with magnetic nanoparticles. This method allows obtaining said nanoparticles in less than 45 minutes, which is surprisingly faster than the methods described in the prior art, and with an encapsulation efficiency of over 90%.

The nanoparticles obtained by this method are biocompatible and biodegradable, particularly useful in the diagnosis and treatment of cancer in humans and other animal species.

All technical and scientific terms used to describe the present invention have the same meaning a person with basic knowledge in the involved technical field understands. However, in order to define the scope of the invention in a clearer manner, a list of the terminology used in this description and the meaning thereof is included hereunder.

"Nanoparticle" should be understood as any particle with sizes in the nanometer scale, preferably with diameters smaller than 500 nm. Additionally, it should be understood that the term "nanoparticle" comprises any configuration it may have, i.e., the term includes nanocapsule or nanosphere, understanding that a nanocapsule consists of a nanoparticle containing a coating that surrounds a center, wherein the molecule of interest is located; and that a nanosphere consists of a microparticle without a coating or defined layers.

"Desolvation agent" should be understood as any chemical compound that induces the formation of aggregates of polymer molecules in solution and subsequent precipitation of these aggregates. This is due to the removal of the solute molecules surrounding the polymer molecules, which causes these polymers to spontaneously associate and form these aggregates.

"Cross-linking agent" should be understood as any chemical compound that has the property of forming covalent or ionic bonds between polymeric chains, whether synthetic or natural polymers. In particular, protein cross-linking agents form covalent bonds between proteins by the reaction of the reactive groups of the cross-linking agent with the functional groups of amino acids (primary amines, sulfhydryls, etc.) forming stable interactions. The term cross-linking agent is also known as "crosslink agent" or "crosslinked agent".

The method of the present invention for obtaining albumin nanoparticles loaded with magnetic nanoparticles comprises a first step (step (a)) of mixing an albumin solution with magnetic nanoparticles.

The albumin used in the present invention can be obtained from any known source such as egg whites (ovalbumin), whey albumin (α-lactalbumin), bovine serum albumin (BSA), human serum albumin (HSA), etc. Although the present method is described for obtaining albumin nanoparticles, this method can be easily adapted for obtaining nanoparticles of any other protein such as gelatin, whey proteins, gliadin, legumin, elastin, zein, soy proteins, casein, α1-globulins, α2-globulins, β-globulins, γ-globulins, among others. In a preferred embodiment, the albumin used in the present invention is human serum albumin.

Magnetic nanoparticles that can be used in the present invention are those comprising one or a mixture of magnetic elements such as iron (Fe), nickel (Ni), cobalt (Co), manganese (Mn), magnesium (Mg), zinc (Zn), gadolinium (Gd), among others. Such nanoparticles can also be composed of the oxides of these elements. For example, nanoparticles that are mainly composed of some iron oxide may contain FeO (iron (II) oxide or wüstite), FeO₂, Fe₃O₄ (iron (II, III) oxide or magnetite), Fe₄O₅, Fe₅O₆, Fe₅O₇, Fe₂₅O₃₂, Fe₁₃O₁₉, Fe₂O₃ (iron (III) oxide), α-Fe₂O₃ (hematite), β-Fe₂O₃, γ-Fe₂O₃ (maghemite), ε-Fe₂O₃, among others. The nanoparticles may also contain ferrites, such as, cobalt ferrite (CoFe₂O₄), manganese ferrite (MnFe₂O₄), nickel ferrite (NiFe₂O₄), lithium ferrite (Li_{0.5}Fe_{2.5}O₄), etc. Ferromagnetic nanoparticles of gold-(Au)-doped iron or zinc/manganese-doped iron oxides (ZnₓMn_{(1 - x)}Fe₃O₄), zinc/iron (ZnₓFe_{(1 - x)}Fe₃O₄), zinc/cobalt (ZnₓCo_{(1 - x)}Fe₃O₄), among others, can also be used. Magnetic nanoparticles of magnetite or maghemite doped with magnesium, manganese, nickel, cobalt, copper, zinc, lanthanides (cerium, gadolinium, terbium, holmium, samarium, europium, etc.), silver, cadmium, etc. can also be used.

In a preferred embodiment of the invention, the magnetic nanoparticles used are iron-oxide nanoparticles of diameters between 5-150 nm. Due to their size, such nanoparticles have superparamagnetic properties (so-called SPIONs), which are of particular interest due to their high biocompatibility and their chemical, physical and magnetic properties, which make them suitable for biomedical applications.

In a preferred embodiment of the present invention, the albumin solution is mixed with the magnetic nanoparticles at a ratio range of 10:1 to 10:5 w/w, preferably at a ratio of 10:2 w/w.

In step (a) of the method of the present invention an ultra-high-speed agitation is performed, which surprisingly allows decreasing the preparation time of these nanoparticles from hours to only minutes. The speed at which the agitation can be performed is between 5,000 and 20,000 rpm, preferably between 8,000 and 20,000 rpm, more preferably between 10,000 and 18,000 rpm. In an even more preferred embodiment of the invention, the stirring rate at this step is 15,000 rpm.

Due to the ultra-high speed at which this step (a) is carried out, only a short stirring time of between 3 to 7 minutes, more preferably between 4 to 6 minutes, is required. In a preferred embodiment of the invention, the stirring time is 5 minutes.

In a preferred embodiment of the present invention, the stirring of step (a) is preferably carried out with a rotor/stator-type homogenizer.

Subsequently, in a preferred embodiment of the invention, the method further comprises a step for adjusting the pH of the mixture of the albumin solution with magnetic nanoparticles to a pH between 9 and 10. Preferably, said adjustment is performed by adding a sufficient amount of base (e.g., NaOH) until the desired pH is achieved.

The method of the present invention comprises a second step (step b), in which a desolvation agent is added dropwise to the previously described mixture of albumin solution and magnetic nanoparticles with constant stirring.

The desolvation agent that can be used in the present invention is selected from the group consisting of acetone, methanol, ethanol, acetonitrile, isopropanol, butanol, or a mixture thereof, salts of polyvalent anions (e.g., SO₄⁻²), salts of polyvalent cations (e.g., Ca²⁺, Mg²⁺), among others, and is not limited to the ones mentioned. Preferably, in the method of the present invention ethanol is used as a desolvation agent.

In a preferred embodiment, the desolvation agent is continuously added to the mixture of albumin solution and magnetic nanoparticles (mixture obtained from step (a)) dropwise, at a rate of from 0.1 to 3 ml/min, preferably at a rate of from 0.1 to 2 ml/min, more preferably at a rate of 1 ml/min. In another preferred embodiment, the desolvation agent is added at a rate of from 0.3 to 0.7 ml per mg of albumin. This range is an approximate value, since the amount of desolvation agent to be added to the mixture shall be sufficient to cause the mixture to become cloudy or whitish.

In a preferred embodiment of the present invention, the albumin solution is mixed with the magnetic nanoparticles at a ratio range of 10:1 to 10:5 w/w, preferably at a ratio of 10:2 w/w.

In step (b) of the method of the present invention an ultra-high-speed agitation is performed, which also allows surprisingly decreasing the preparation time of these nanoparticles from hours to only minutes. The speed at which the agitation can be performed is between 5,000 and 20,000 rpm, preferably between 8,000 and 20,000 rpm, more preferably between 10,000 and 18,000 rpm. In an even more preferred embodiment of the invention, the stirring rate at this step is 15,000 rpm.

Due to the ultra-high-speed at which this step (b) is carried out, only a short stirring time of not more than 15 minutes is required. Preferably, the stirring time in each of these steps is between 1 to 15 minutes, more preferably between 2 to 10 minutes, even more preferably between 2 to 8 minutes. In a preferred embodiment of the invention, the stirring time is 5 minutes.

In a preferred embodiment of the present invention, the stirring of step (b) is preferably carried out with a rotor/stator-type homogenizer.

The method of the present invention comprises a third step (step (c), in which a cross-linking agent is added to the previously-described mixture obtained from step (b) with constant stirring.

The cross-linking agent which may be used in the present invention is selected from the group consisting of homo- or heterobifunctional agents with identical or different reactive groups, respectively. The functional groups may be aryl azide, carbodiimide, hydrazide, hydroxymethyl phosphine, imidoester, isocyanate, carbonyl, maleimide, NHS-ester, PFP-ester, psoralen, pyrimidyl disulfide, vinyl sulfone.

Cross-linking agents that can be used in the present method are homobifunctional such as glutaraldehyde, diaminoalkanes, di(N-succinimidyl) carbonate, di(N-succinimidyl) suberate, dimethyl adipimate (DMA), dimethyl pimelimidate (DMP), dimethyl suberimidate (DMS), bis(sulfosuccinimidyl) suberate (BSSS, BS3), disuccinimidyl glutarate (DSG), ethylene glycolbis(sulfosuccinimidylsuccinate) (sulfo-EGS), disuccinimidyl suberate (DSS), dithiobis(succinimidyl propionate) (DTSP, Lomant's reagent), ethylene glycolbis(succinimidylsuccinate) (EGS), bis(sulfosuccinimidyl) glutarate (BS2G), 3,3'-dithiobis(sulfosuccinimidylpropionate) (DTSSP), disuccinimidyl tartrate (DST), Bis(2-(2-(succinimidooxycarbonyloxy]ethyl)sulfone (BSOCOES), 1,4-di-(3'- (2'pyridyldithio)-propionamide) butane (DPDPB), sulfodisuccinimidyl tartrate (sulfo DST), dithiobis(succinimidyl propionate) (DSP), ethylene glycol bis(succinimidyl succinate) (EGS); heterobifunctional agents such as succinimidyl-4-[Nmaleimidomethyl]cyclohexane-I-carboxylate (SMCC), SANPAH, n- sulfosuccinimidyl-6-[4'-azido-2'-nitrophenylamino] hexanoate (sulfo-SANPAH), m- maleimidobenzoyl-N-hydroxysuccinimide ester (MBS), m-maleimidobenzoyl-N- hydroxysulfosuccinimide ester (sulfo-MBS), N-γ-maleimidobutyryloxylysuccinimide ester (GMBS), N-γ-maleimidobutyryloxylysulfosuccinimide ester (GMBS sulfo), N- (8-maleimidocaproic azido) hydrazide (EMCH), N-(8-maleimidocaproyloxy) succinimide ester (EMCS), N-(ε-maleimidocaproyloxy) sulfo succinimide ester (EMCS sulfo), N-(p-maleimidophenyl) isocyanate (PMPI), N-succinimidyl(4- iodoacetyl)aminobenzoate (SlAB), succinimidyl 3-(2-pyridyldithio)propionate (SPDP), succinimidyl 6-[3-[3(2-pyridyldithio)propionamide] hexanoate (LC-SPDP), N-succinimidyl bromoacetate (SBA), N-[e-maleimidocaproyloxy] succinimide ester (EMCS), succinimidyl-6-[B-maleimidopropionamide] hexanoate (SMPH), sulfosuccinimidyl 6-(3'-[2-pyridyldithio]-propionamide) hexanoate (sulfo-LC-SPDP), N-succinimidyl 4-[4-maleimidophenyl] butyrate (SMPB), (3-[2-[2- Pyridyldithio]propionyl hydrazide) (PDPH), N-succinimidyl iodoacetate (SIA), N-(B-maleimidopropyloxy)succinimide ester (BMPS) and N-5-azido-2-nitrobenzoyloxy succinimide (ANB-NOS); among others, without being limited to the mentioned cross-linking agents. Preferably, in the method of the present invention glutaraldehyde is used as cross-linking agent. In a preferred embodiment, 10 µl of a 6.25% glutaraldehyde solution is added per mg of albumin.

In step (c) of the method of the present invention an ultra-high-speed agitation is performed, which also allows surprisingly decreasing the preparation time of these nanoparticles from hours to only minutes. The speed at which the agitation can be performed is between 5,000 and 20,000 rpm, preferably between 8,000 and 20,000 rpm, even more preferably between 10,000 and 18,000 rpm. In an even more preferred embodiment of the invention, the stirring rate is 15,000 rpm at this step.

Due to the ultra-high-speed at which this step (c) is carried out, only a short stirring time of not more than 15 minutes is required. Preferably, the stirring time in each of these steps is between 1 to 15 minutes, more preferably between 2 to 10 minutes, even more preferably between 2 to 8 minutes. In a preferred embodiment of the invention, the stirring time is 5 minutes.

In a preferred embodiment of the present invention, the stirring of step (c) is preferably carried out with a rotor/stator-type homogenizer.

The present invention includes a fourth step (step d), which comprises obtaining the albumin nanoparticles loaded with magnetic nanoparticles from the mixture of step (c). The diameter of the nanoparticles obtained by this rapid method is between 30 to 300 nm, preferably between 40 to 120 nm, even more preferably between 60 to 100 nm.

In some preferred embodiments of the invention, the method may include other additional steps to the previously-mentioned ones. For example, in a preferred embodiment of the present invention the step of obtaining the nanoparticles (step (d) may additionally include a washing step of said nanoparticles, which may preferably be performed by one or multiple washes with distilled water or phosphate buffered saline (PBS). In another preferred embodiment of the invention, the step of obtaining the nanoparticles (step d) may additionally include a centrifugation step at a speed between 8,000 to 20,000 g, preferably between 10,000 to 15,000 g.

All the steps of the method of the present invention are carried out at room temperature (between 20-25°C).

The following are examples of embodiments of the invention, which have been included for the purpose of illustrating the invention, the preferred embodiments and comparative examples thereof, but in no case should they be considered to restrict the scope of the patent application, which is only delimited by the contents of the attached claims.

### EXECUTION EXAMPLES

### Example 1: Characterization of superparamagnetic iron-oxide nanoparticles (SPIONs)

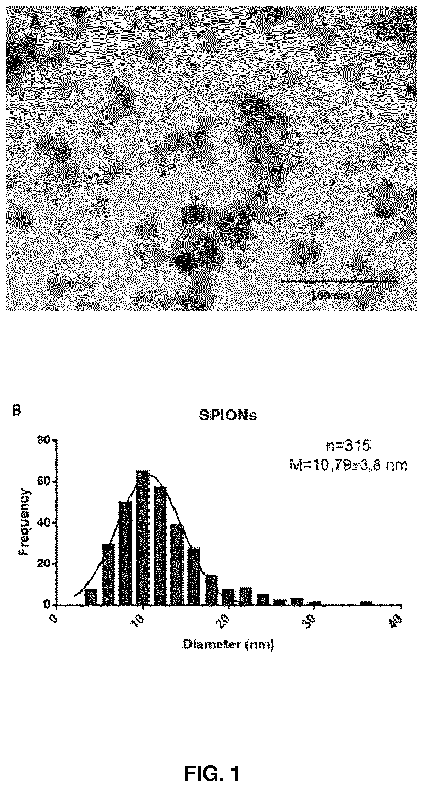

SPIONs (n = 315) were analyzed with a transmission electron microscope (TEM) to determine their size and morphology. As seen in FIG. 1A, the nanoparticles showed a mostly spherical morphology with an average diameter of 10.79 ± 3.8 nm (FIG. 1B).

### Example 2: Method for the synthesis of albumin nanoparticles (NPs)

Albumin nanoparticles were prepared using the conventional desolvation method to compare them with the albumin nanoparticles obtained if the stirring rate is modified at the time of adding the desolvation agent.

For this purpose, 10 ml of a 10 mg/ml solution of albumin in distilled water was previously prepared on a magnetic stirrer at 800 rpm for 30 min. Then, 1 ml of this solution was placed in a 5 ml beaker and 50 µl of a 0.1 M NaOH solution was added and instead of using the magnetic stirrer that would be used in the conventional method, a rotor/stator-type homogenizer was used, and the solution was stirred at a speed of 10,000 rpm for 5 min. After this time the pH was measured with indicator strips and the value obtained was 9. Then, under constant agitation at 10,000 rpm, 3 to 4 ml of ethanol was added dropwise until the solution turned whitish, indicating the formation of nanoparticles.

The albumin nanoparticles obtained by both methods were analyzed by transmission electron microscopy (TEM), and their hydrodynamic size was measured by dynamic light scattering (DLS).

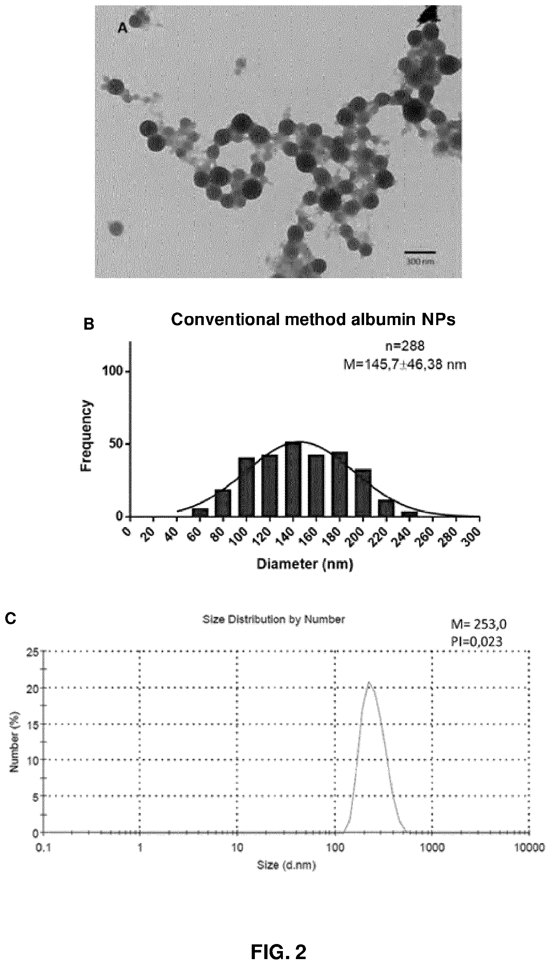

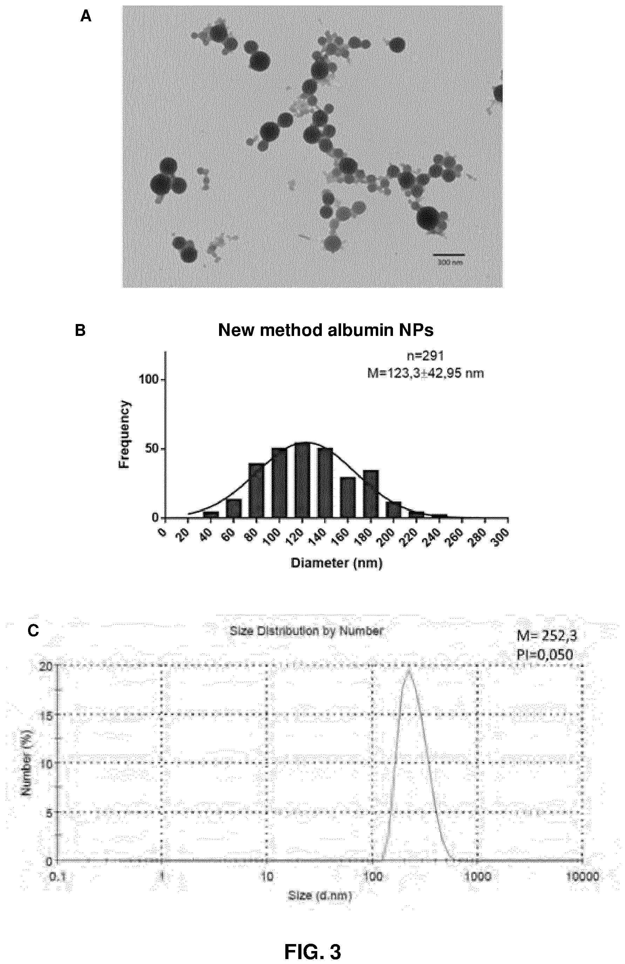

The morphology of the albumin nanoparticles obtained with the conventional method (FIG. 2A) and the morphology of the albumin nanoparticles obtained with the rapid method (FIG. 3A) are highly similar. In turn, the average diameter of the nanoparticles obtained with the conventional method (FIG. 2B) was close to 146 nm (n = 288) while the diameter of the nanoparticles obtained with the rapid method (FIG. 3B) was close to 123 nm (n = 291). Similarly, the hydrodynamic size of the nanoparticles obtained with the conventional method (FIG. 2C) was close to 253 nm, and the hydrodynamic size of the nanoparticles obtained with the rapid method (FIG. 3C) was close to 252.3 nm.

These results show that albumin nanoparticles obtained by both methods are similar in both morphology and size, thus validating the first step of this rapid method.

### Example 3: Development of albumin nanoparticles loaded with SPIONs nanoparticles

After verifying that the synthesis of albumin nanoparticles with the homogenizer was feasible, the method for the encapsulation of SPIONs in albumin nanoparticles was standardized. For this purpose, three trials were carried out, in which different concentrations of SPIONs (1, 2 and 3 mg) were added to the albumin solution.

50 ml of an albumin solution at 10 mg/ml in distilled water was prepared under agitation at 800 rpm on a magnetic stirrer for 30 min. Then 1 ml of this solution was placed in a 5 ml beaker and 1, 2 or 3 mg of SPIONs was/were added and this mixture was stirred for 5 min at 15,000 rpm. Then, the pH was adjusted to 9 by adding 50 µl of 0.1 M NaOH solution and stirred for 5 min.

Then, 3 to 6 ml of absolute ethanol was added dropwise continuously at a rate of approximately 1 ml/min and under agitation at 10,000 rpm until the solution turned whitish, indicating the formation of nanoparticles.

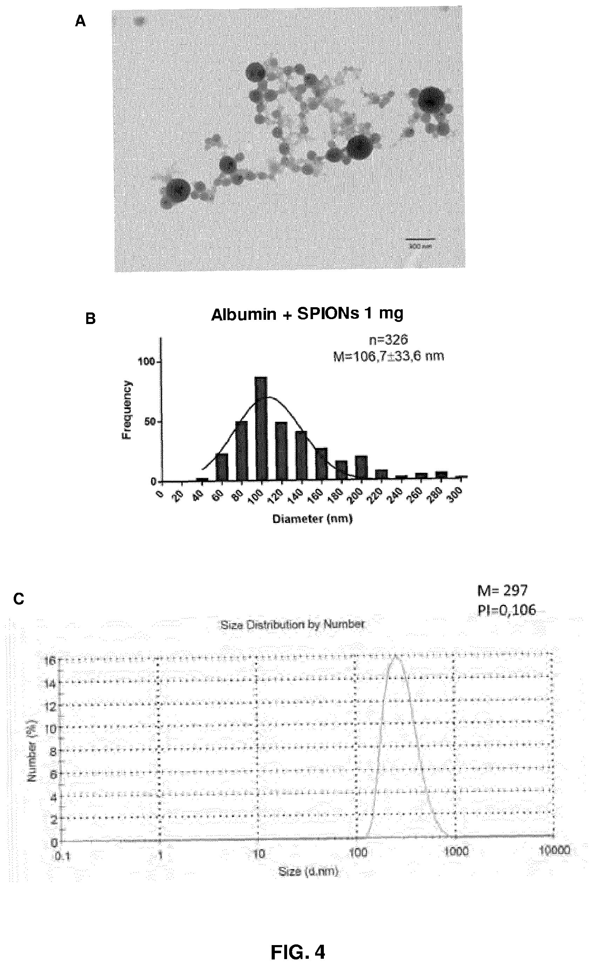

FIG. 4 shows the results of nanoparticles that were formed by adding 1 mg of SPIONs. FIG. 4A shows the morphology of the albumin nanoparticles loaded with SPIONs. The average diameter of these nanoparticles was 106.7 ± 33.6 nm (n = 326) as seen in FIG. 4B and their average hydrodynamic size was close to 297 nm (FIG. 4C).

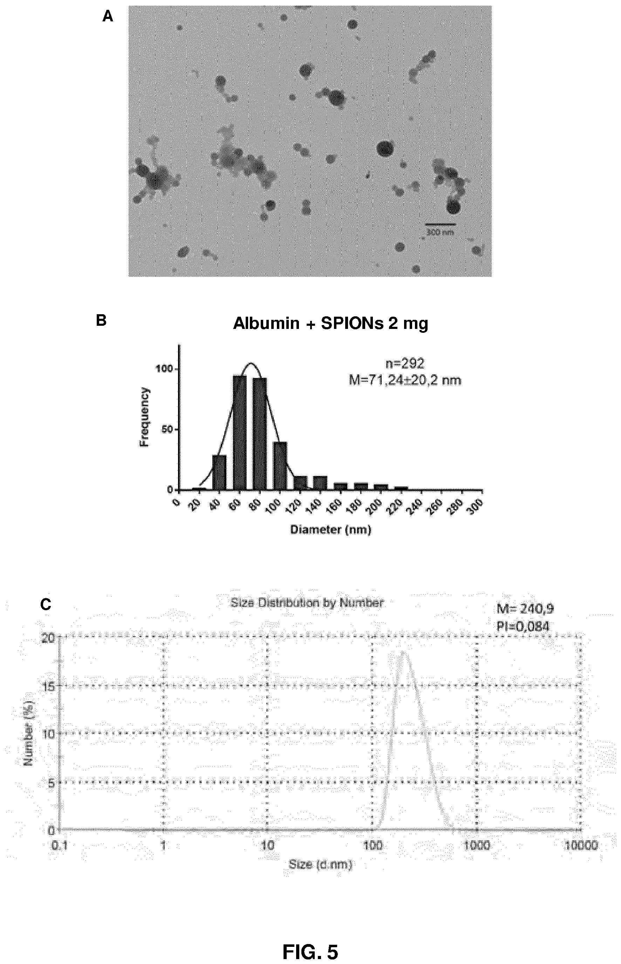

FIG. 5 shows the results of nanoparticles that were formed by adding 2 mg of SPIONs. FIG. 5A shows the morphology of the albumin nanoparticles loaded with SPIONs. The average diameter of these nanoparticles was 71.24 ± 20.2 nm (n = 292) as seen in FIG. 5B and their average hydrodynamic size was close to 240.9 nm (FIG. 4C).

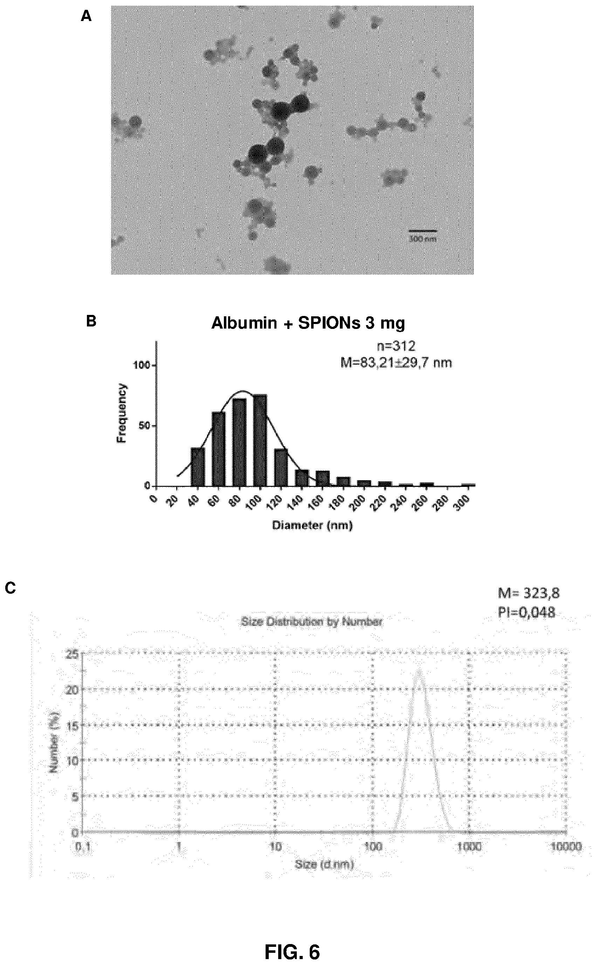

FIG. 6 shows the results of nanoparticles that were formed by adding 3 mg of SPIONs. FIG. 6A shows the morphology of the albumin nanoparticles loaded with SPIONs. The average diameter of these nanoparticles was 83.21 ± 29.7 nm (n = 312) as seen in FIG. 6B and their average hydrodynamic size was close to 323.8 nm (FIG. 6C).

These results show that the presence of SPIONs affects the formation of albumin nanoparticles, as it decreases the diameter and the proportion of nanoparticles formed. Since the concentration of 2 mg of SPIONs presented the lowest dispersion values, this concentration was selected for the following experiments.

### Example 4: Optimization of the stirring time of the albumin solution and SPIONs when the desolvation agent is added

The encapsulation efficiency was analyzed as a function of the stirring time of the albumin and SPIONs solution when the desolvation agent was added. For this purpose, 1 ml of an albumin solution at 10 mg/ml was taken and 2 mg of SPIONs were added to this solution, and the pH was adjusted to 9. 3 to 4 ml of absolute ethanol was then added dropwise continuously and under agitation at 10,000 rpm for 2 to 5 minutes.

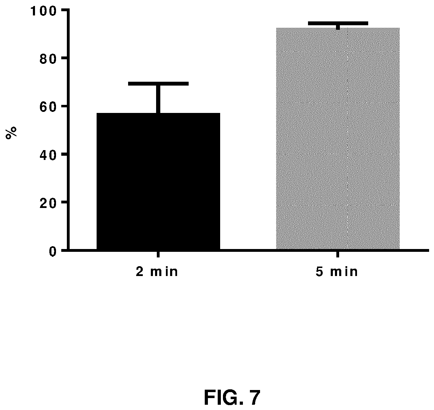

FIG. 7 shows the results of this assay, in percentage of nanoparticles loaded with SPIONs. The average values were 56.3 ± 5.8 and 91.7 ± 1.0 for stirring times of 2 and 5 min, respectively. In both experiments, albumin nanoparticles loaded with SPIONs were obtained. Higher encapsulation efficiency was observed with a 5 min stirring time.

### Example 5: Optimization of stirring rate and pH of albumin solution and SPIONs when desolvation agent is added

Since the dispersion and size of the albumin nanoparticles change due to the effect of the SPIONs, the stirring rate and pH parameters were optimized with the objective of having nanoparticles close to 100 nm and with low dispersion, maintaining the encapsulation efficiency equal to or greater than 90%. The synthesis of albumin nanoparticles with and without SPIONs was evaluated at 15,000 and 20,000 rpm, at pH 9 and 10. In those experiments of albumin nanoparticles with SPIONs, 2 mg of SPIONs were added to the solutions. The stirring time in each experiment was 5 min.

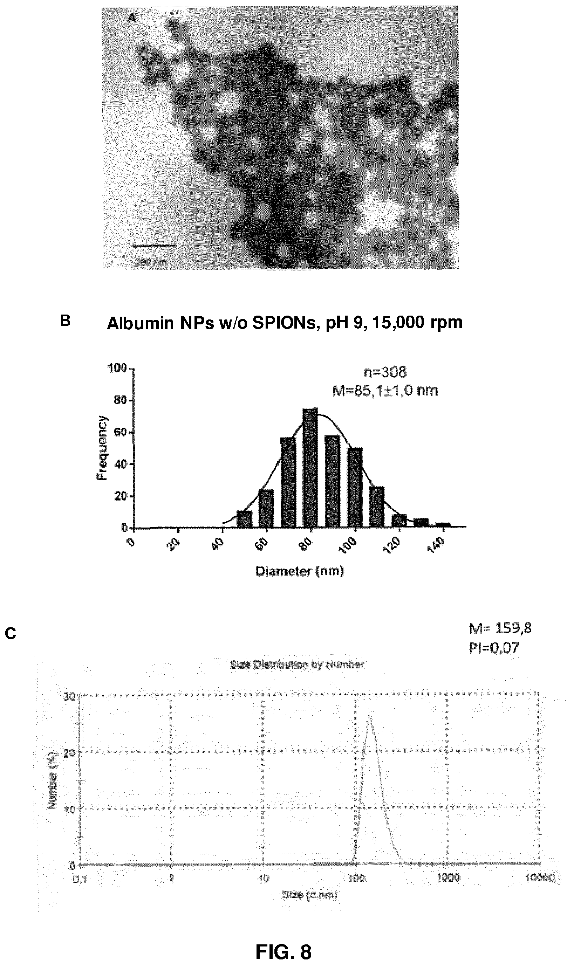

The results of these assays are shown in FIGs 8-13. FIG. 8A shows the morphology of albumin nanoparticles without SPIONs, synthesized at pH 9, and a stirring rate of 15,000 rpm. The average diameter of these nanoparticles was 85.1 ± 1.0 nm (n = 308) as seen in FIG. 8B, and their average hydrodynamic size was 159.8 nm (FIG. 8C).

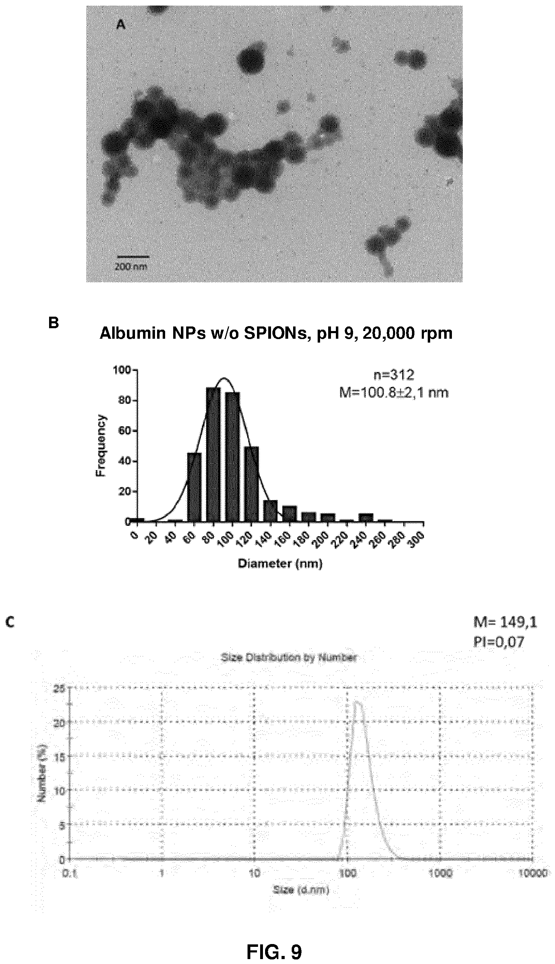

FIG. 9A shows the morphology of albumin nanoparticles without SPIONs, synthesized at pH 9, and a stirring rate of 20,000 rpm. The average diameter of these nanoparticles was 100.8 ± 2.1 nm (n = 312) as seen in FIG. 9B, and their average hydrodynamic size was 149.1 nm (FIG. 9C).

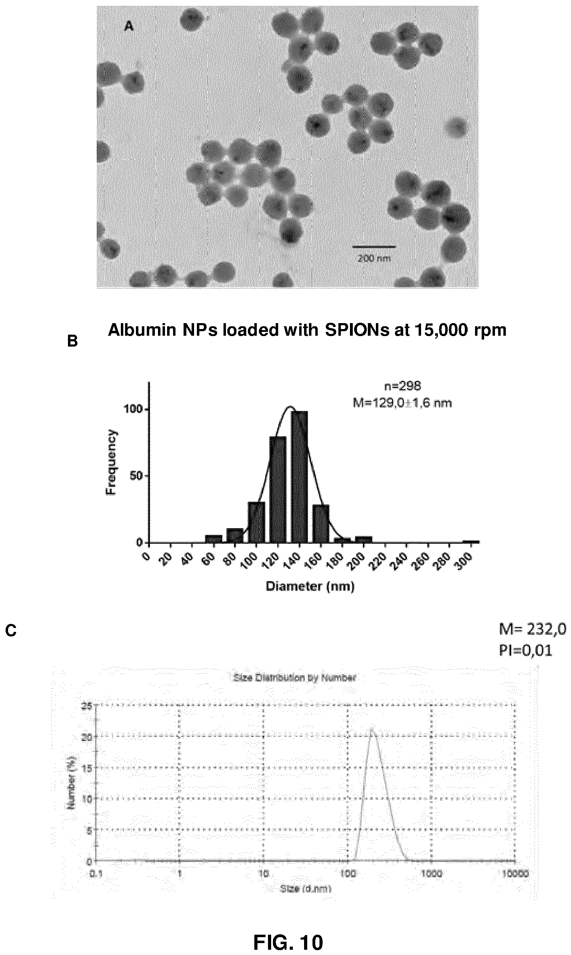

FIG. 10A shows the morphology of albumin nanoparticles loaded with SPIONs, synthesized at pH 9, and a stirring rate of 15,000 rpm. The average diameter of these nanoparticles was 129.0 ± 1.6 nm (n = 298) as seen in FIG. 10B, and their average hydrodynamic size was 232.0 nm (FIG. 10C).

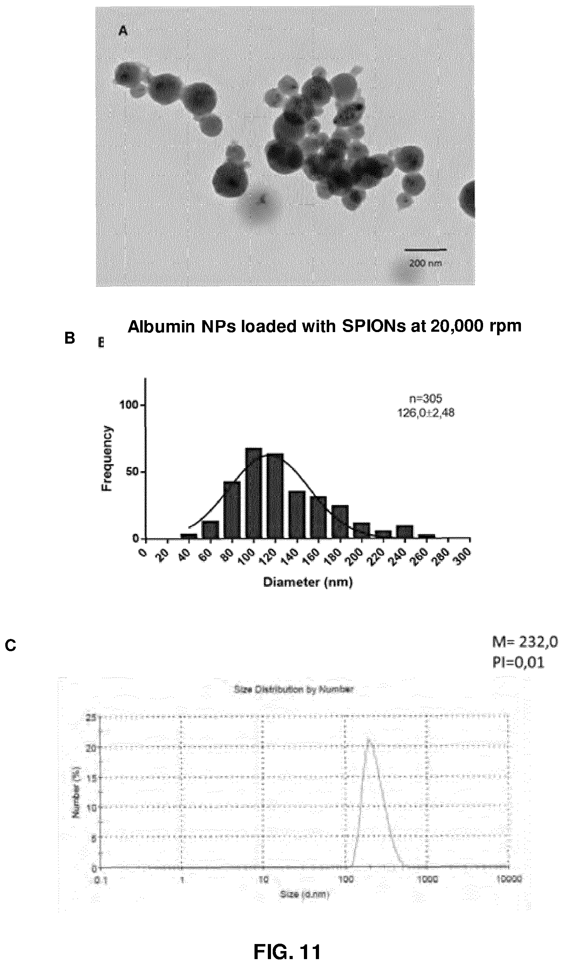

FIG. 11A shows the morphology of albumin nanoparticles loaded with SPIONs, synthesized at pH 9, and a stirring rate of 20,000 rpm. The average diameter of these nanoparticles was 126.0 ± 2.48 nm (n = 305) as seen in FIG. 11B, and their average hydrodynamic size was 232.0 nm (FIG. 11C).

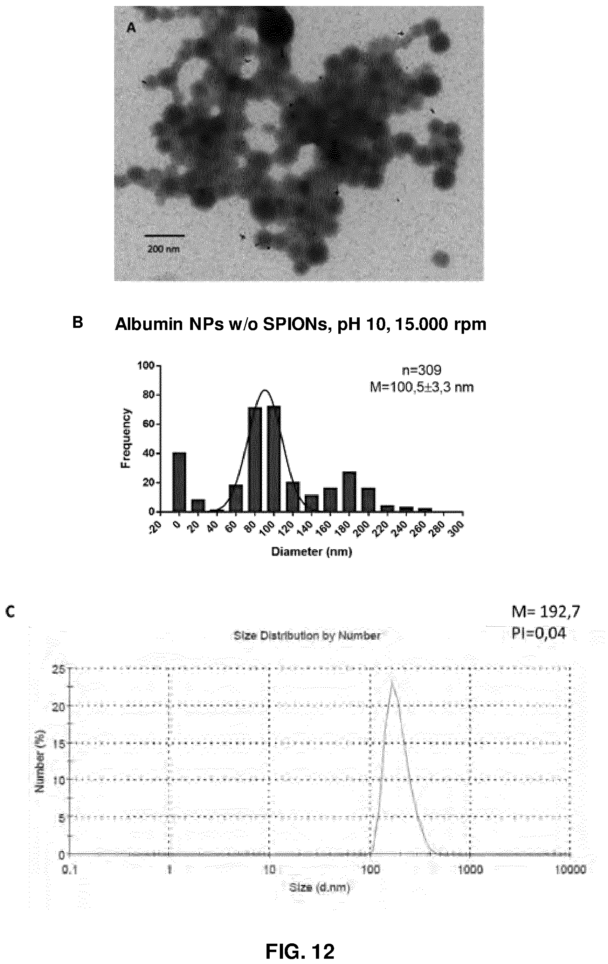

FIG. 12A shows the morphology of albumin nanoparticles without SPIONs, synthesized at pH 10, and a stirring rate of 15,000 rpm. The average diameter of these nanoparticles was 100.0 ± 3.3 nm (n = 309) as seen in FIG. 12B, and their average hydrodynamic size was 192.7 nm (FIG. 12C).

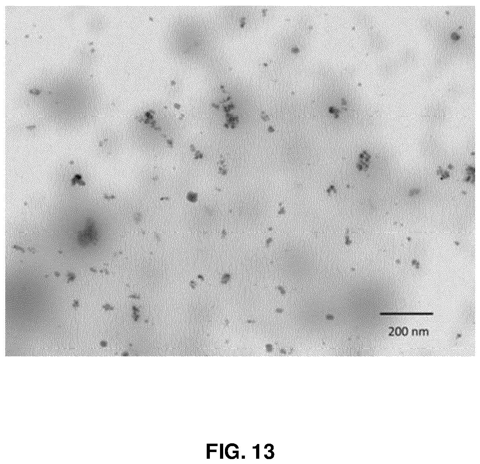

FIG. 13 shows the result of the synthesis of albumin nanoparticles loaded with SPIONs, at pH 10, and stirring rate at 20,000 rpm. The electron microscopy image shows that there was no nanoparticle formation at this pH value.

Thus, the smallest nanoparticle diameter is achieved at a speed of 15,000 rpm at pH 9, both for albumin nanoparticles with or without SPIONs (FIG. 8 and 10), compared to those obtained at 20,000 rpm (FIG. 9 and 11). Increasing the pH did not contribute to improve the morphology or size of the nanoparticles (FIG. 12 and 13). In summary, the optimal synthesis of albumin nanoparticles with SPIONs was obtained using 2 mg of SPIONs per 10 mg of albumin, a previous stirring time of 5 min at 15,000 rpm at pH 9.

### Example 6: Analysis of albumin nanoparticles loaded with SPIONs stabilized with the cross-linking agent at different pH values

The stability of albumin nanoparticles loaded with SPIONs was analyzed after incubating the same in acidic solutions of different pH.

Two groups of SPIONs-loaded albumin nanoparticles were used, both prepared by the above method with the optimal synthesis parameters, but to one of the groups, after addition of ethanol, 100 µl of a 6.25% ethanolic solution of glutaraldehyde was added and stirred for an additional 5 min at 15,000 rpm. Subsequently, both groups of albumin nanoparticles loaded with SPIONs were centrifuged at 10,000 g for 10 min, washed 2 times with distilled water and resuspended in citrate buffer at pH 2, 3, 4, 5 and 6. Then, TEM microscopy observations were performed after 24, 48, and 72 h of incubation in citrate buffer.

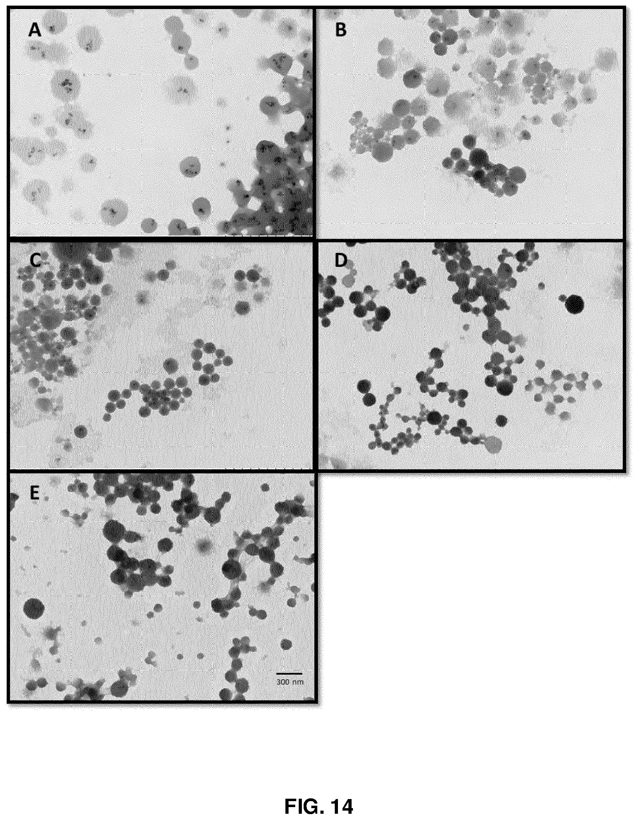

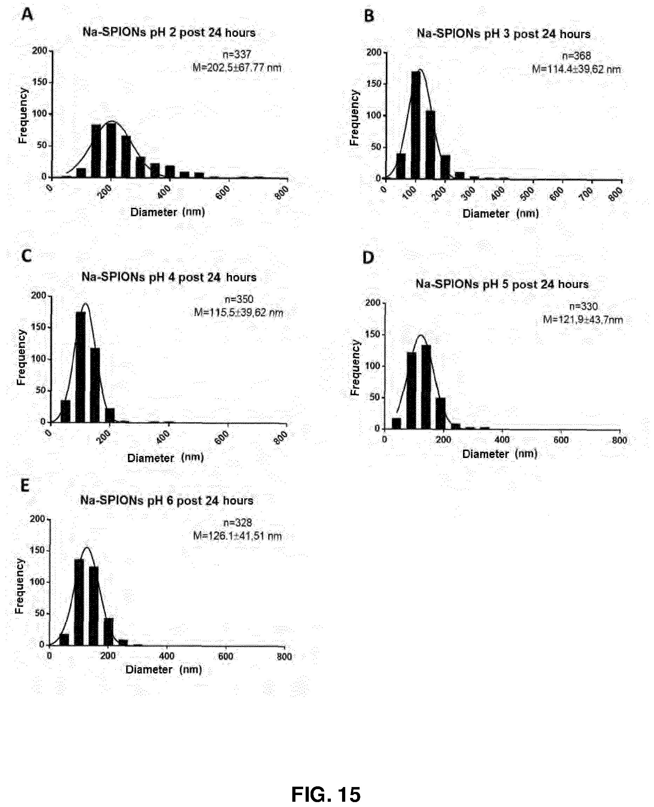

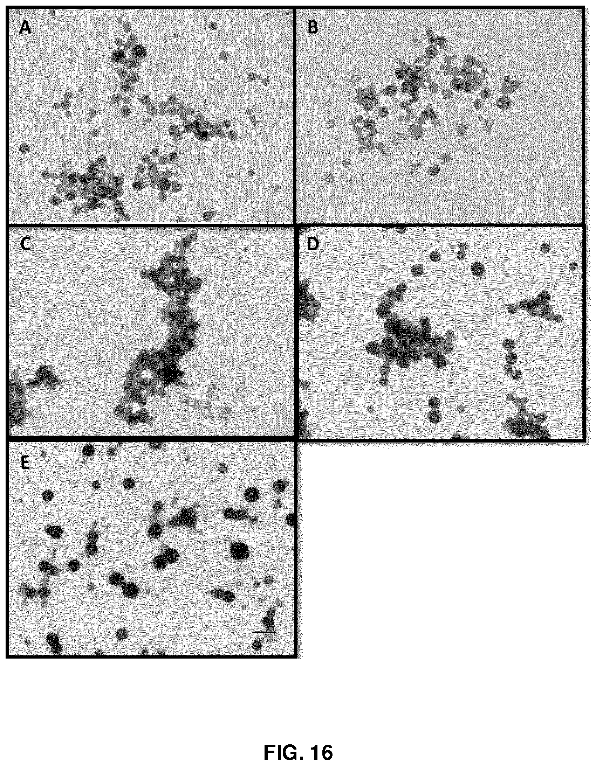

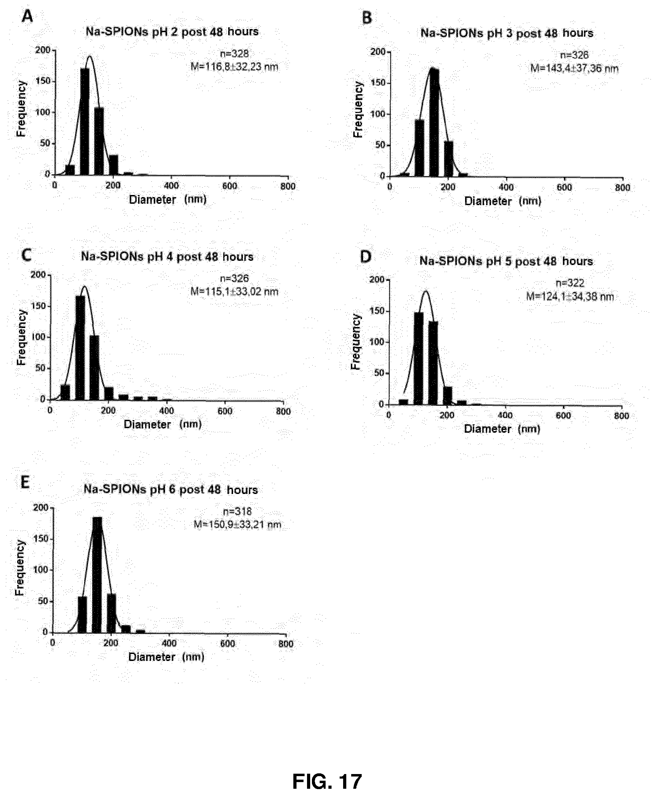

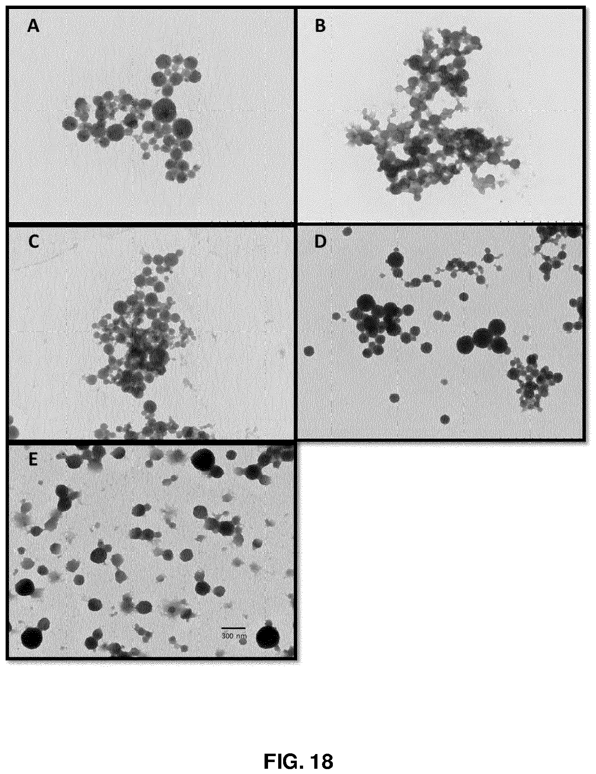

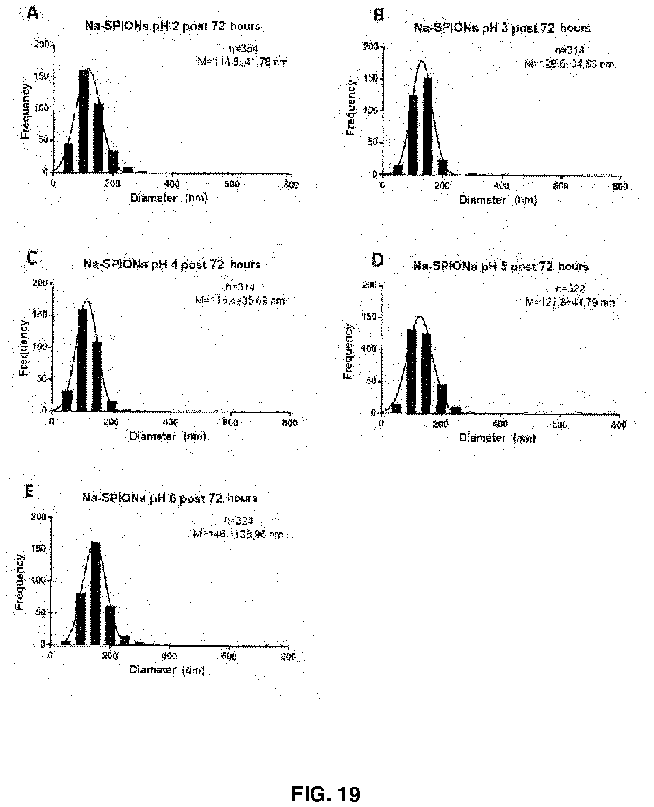

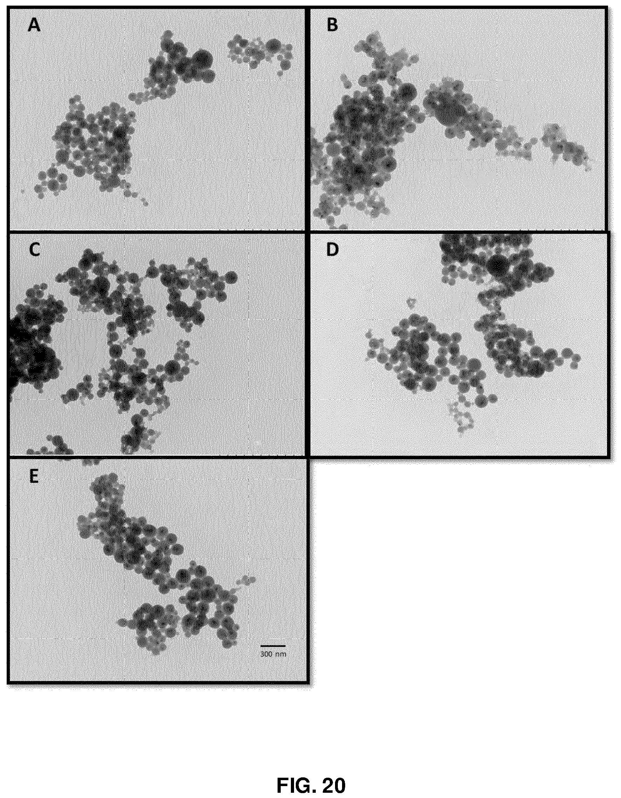

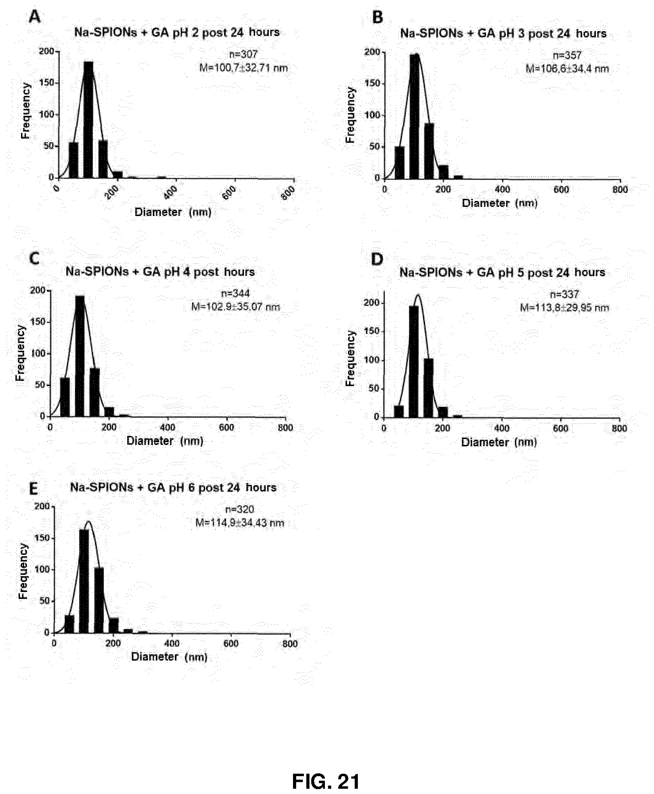

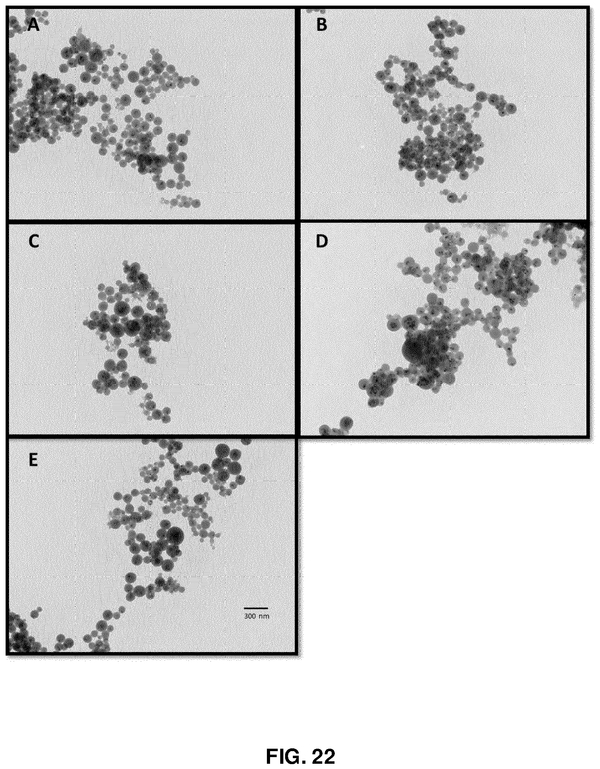

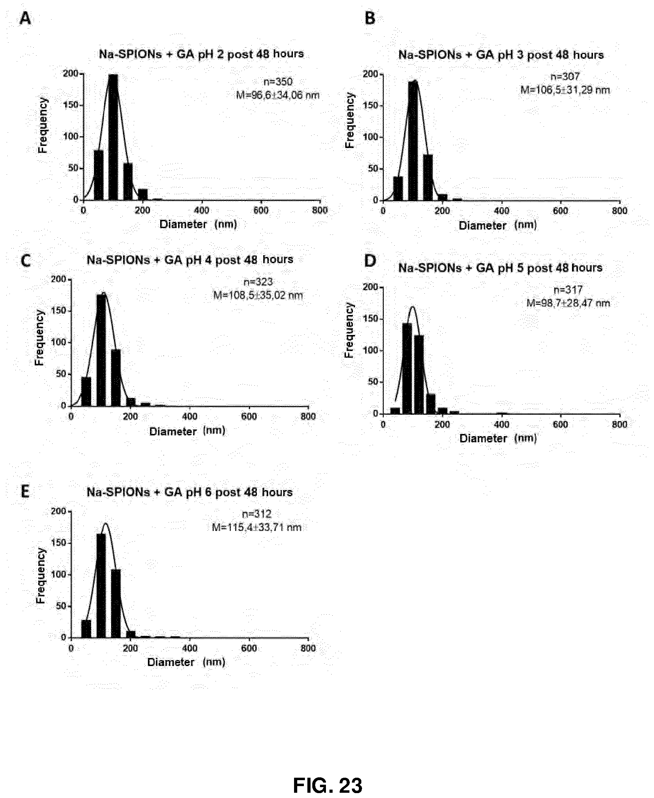

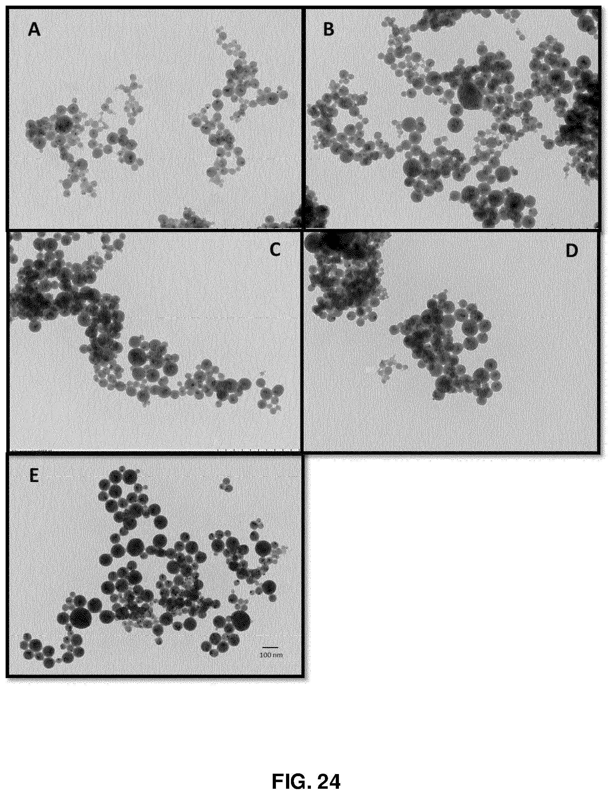

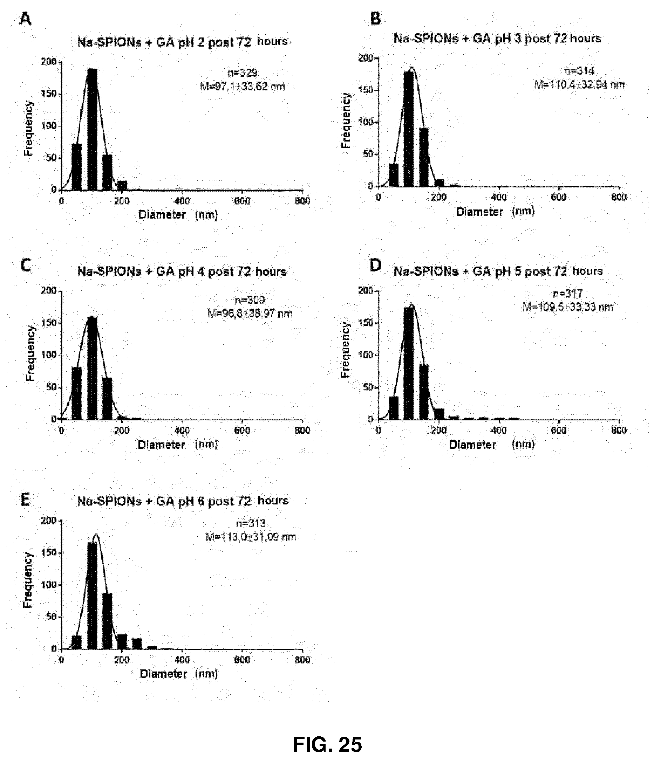

The analysis of the images obtained by TEM show changes in the morphology, structure and size of the albumin nanoparticles loaded with SPIONs obtained by the standard method without adding glutaraldehyde (FIG. 14-19). The nanoparticles showed greater structural changes at pH 2, where disintegrating nanoparticles were observed (FIG. 14A, 16A, 18A) and high variability in size in those nanoparticles that were incubated for 24 hours (FIG. 15A, 17A, 18A). At pH 3, 4, 5 and 6, the albumin nanoparticles loaded with SPIONs showed diffuse contours and increased permeability to the dye uranyl acetate used to give contrast to the samples, making them appear darker (FIG. 14B-E, 16B-E, 18B-E), but showed similar size dispersion in all groups analyzed (FIG. 15B-E, 17B- E, 19B-E). On the other hand, albumin nanoparticles loaded with glutaraldehyde- stabilized SPIONs showed no changes in morphology, structure (FIG. 20A-E, 22A- E and 24A-E) or size (FIG. 21A-E, 23A-E and 25A-E) for any pH value and treatment time. Glutaraldehyde is shown to be useful in providing stability to albumin nanoparticles in solutions with acidic pH values for up to 72 hours.

## Claims

1. A method for obtaining albumin nanoparticles loaded with magnetic nanoparticles, **CHARACTERIZED in that** it comprises the steps of:
a) mixing an albumin solution with magnetic nanoparticles and stirring the mixture at 8,000 to 20,000 rpm for 3 to 7 minutes;
b) dropwise adding a desolvation agent to the mixture of step (a) while maintaining a constant agitation at a speed between 8,000 and 20,000 rpm for 1 to 15 minutes;
c) adding a cross-linking agent to the mixture of step (b) while maintaining a constant agitation at a speed between 8,000 and 20,000 rpm for 1 to 15 minutes, and
d) obtaining albumin nanoparticles loaded with magnetic nanoparticles from the mixture of step (c).

2. The method according to claim 1, **CHARACTERIZED in that** the magnetic nanoparticles are iron-oxide nanoparticles.

3. The method according to claim 2, **CHARACTERIZED in that** the magnetic nanoparticles are superparamagnetic iron-oxide nanoparticles (SPIONs).

4. The method according to claim 1, **CHARACTERIZED in that** the stirring of step (a) is performed at a speed of 15,000 rpm for 5 minutes.

5. The method according to claim 1, **CHARACTERIZED in that** the stirring of step (b) is performed at a speed of 15,000 rpm for 5 minutes.

6. The method according to claim 1, **CHARACTERIZED in that** the stirring of step (c) is performed at a speed of 15,000 rpm for 5 minutes.

7. The method according to claim 1, **CHARACTERIZED in that** the stirring of step (a) is performed with a rotor/stator-type homogenizer.

8. The method according to claim 1, **CHARACTERIZED in that** the stirring of step (b) is performed with a rotor/stator-type homogenizer.

9. The method according to claim 1, **CHARACTERIZED in that** the stirring of step (c) is performed with a rotor/stator-type homogenizer.

10. The method according to claim 1, **CHARACTERIZED in that** the albumin solution and the magnetic nanoparticles are mixed at a ratio between 10:1 to 10:5 w/w.

11. The method according to claim 1, **CHARACTERIZED in that** it further comprises adjusting the pH of the step mixture to between 9 and 10.

12. The method according to claim 1, **CHARACTERIZED in that** the desolvation agent is selected from the group consisting of ethanol, methanol, isopropanol, and acetone, as well as a combination thereof.

13. The method according to claim 1, **CHARACTERIZED in that** the desolvation agent is added to the mixture of albumin solution and magnetic nanoparticles at a rate of from 0.1 to 2 ml/min.

14. The method according to claim 1, **CHARACTERIZED in that** the desolvation agent is added at a rate of from 0.3 to 0.7 ml per mg of albumin.

15. The method according to claim 1, **CHARACTERIZED in that** the cross-linking agent is homobifunctional.

16. The method according to claim 15, **CHARACTERIZED in that** the homobifunctional cross-linking agent is glutaraldehyde.

17. The method according to claim 16, **CHARACTERIZED in that** the glutaraldehyde has a concentration at 6.25% and is added at a rate of 10 µl per mg albumin.

18. The method according to claim 1, **CHARACTERIZED in that** the albumin nanoparticles loaded with magnetic nanoparticles have a diameter of from 30 to 300 nm.

19. The method according to claim 1, **CHARACTERIZED in that** step (d) further comprises a step of washing the albumin nanoparticles loaded with magnetic nanoparticles.

20. The method according to claim 19, **CHARACTERIZED in that** the washing step is performed with a solution, which is selected from distilled water and saline phosphate buffer.

21. The method according to claim 1, **CHARACTERIZED in that** step (d) further comprises a step of centrifuging the albumin nanoparticles loaded with magnetic nanoparticles.

22. The method according to claim 21, **CHARACTERIZED in that** the centrifugation step is carried out at a speed between 8,000 to 20,000 g.
